# EUROPÄISCHE PATENTSCHRIFT

(11) **EP 0 882 048 B1**
(45) Veröffentlichungstag und Bekanntmachung des Hinweises auf die Patenterteilung: **04.05.2005**
(21) Anmeldenummer: 97901082.4
(22) Anmeldetag: 24.01.1997
(51) Int. Cl.: C07D 471/04, A61K 31/44

(54) **3-ALKYLIMIDAZOPYRIDINE**
3-ALKYLIMIDAZOPYRIDINES
3-ALKYLIMIDAZOPYRIDINES

(30) Priorität: 26.01.1996 DE 19602855; 07.02.1996 EP 96101728
(43) Veröffentlichungstag der Anmeldung: 09.12.1998
(73) Patentinhaber: ALTANA Pharma AG, 78467 Konstanz (DE)
(72) Erfinder: RAINER, Georg, D-78464 Konstanz (DE); SIMON, Wolfgang-Alexander, D-78464 Konstanz (DE); RIEDEL, Richard, D-88339 Bad Waldsee (DE); POSTIUS, Stefan, D-78467 Konstanz (DE); GRUNDLER, Gerhard, D-78464 Konstanz (DE); SENN-BILFINGER, Jörg, D-78464 Konstanz (DE)
(86) Internationale Anmeldenummer: PCT/EP1997/000333
(87) Internationale Veröffentlichungsnummer: WO 1997/027192

(56) Entgegenhaltungen:
- EP-A- 0 033 094
- EP-A- 0 204 285
- EP-A- 0 268 989
- EP-A- 0 308 917
- WO-A-94/18199
- WO-A-95/10518
- WO-A-96/03403
- WO-A-96/03404
- DATABASE WPI Week 9105 Derwent Publications Ltd., London, GB; AN 90-373004 XP002009764 & JP 02 270 873 A (FUJISAWA PHARM. CO., LTD.)

## Beschreibung

### Anwendungsgebiet der Erfindung

Die Erfindung betrifft neue 3-Alkylimidazopyridine, die in der pharmazeutischen Industrie als Wirkstoffe für die Herstellung von Arzneimitteln verwendet werden soilen.

### Bekannter technischer Hintergrund

In der europäischen Patentanmeldung EP-A-0 033 094 werden Imidazo[1,2-a]pyridine beschrieben, die in 8-Position einen Arylsubstituenten tragen, der bevorzugt ein Phenyl-, Thienyl-, Pyridyl- oder ein durch Chlor, Fluor, Methyl, tert.-Butyl, Trifluormethyl, Methoxy oder Cyan substituierter Phenylrest ist. Als besonders interessante Arylreste sind in der EP-A-0 033 094 die Reste Phenyl, o- oder p-Fluorphenyl, p-Chlorphenyl und 2,4,6-Trimethylphenyl genannt, wovon die Reste Phenyl, o- oder p-Fluorphenyl und 2,4,6-Trimethylphenyl besonders bevorzugt sind. - in den europäischen Patentanmeldungen EP-A-0 204 285, EP-A-0 228 006, EP-A-0 268 989 und EP-A-0 308 917 werden Imidazo[1,2-a]pyridine beschrieben, die in 3-Position einen ungesättigten aliphatischen Rest, insbesondere einen (substituierten) Alkinylrest tragen. - In der europäischen Patentanmeldung EP-A-0 266 890 werden Imidazo[1,2-a]pyridine beschrieben, die in 8-Position durch einen Alkenyl-, Alkyl- oder Cycloalkylalkylrest substituiert sind.

### Beschreibung der Erfindung

Es wurde nun gefunden, daß die nachfolgend näher beschriebenen Verbindungen, die sich von den Verbindungen des Standes der Technik insbesondere durch die Substitution in 3- oder in 8-Position unterscheiden, überraschende und besonders vorteilhafte Eigenschaften besitzen.

Gegenstand der Erfindung sind Verbindungen der Formel I (siehe beigefügtes Formelblatt), worin
- R0: 1-4C Alkyl bedeutet,
- R1: 1-4C-Alkyl bedeutet,
- R2: 1-4C-Alkyl bedeutet,
- R3: SO₂-R6,CO-R7 oder COO-R8 bedeutet,
- A: NH bedeutet,
- R6: 1-4C-Alkyl bedeutet,
- R7: durch R16 substituiertes Phenyl bedeutet,
- R8: durch R11 substituiertes 2-4C-Alkyl, durch R12 substituiertes 1-4C-Alkyl, -N=C(R13)R14 oder durch R15 substituiertes 2-4C-Alkyl bedeutet,
- R11: 1-4C-Alkoxy-1-4C-alkoxy bedeutet,
- R12: 1-4C-Alkoxycarbonyl, Carboxy, Thienyl, Pyridyl oder durch R16 substituiertes Phenyl bedeutet,
- R13: 1-4C-Alkyl und
- R14: 1-4C-Alkyl bedeuten,
- R15: Phthalimidyl oder -S(O)ₙ-R19 bedeutet, wobei
- R16: Hydroxy oder Benzoyl bedeutet,
- R19: 1-4C-Alkyl bedeutet und
- n: die Zahlen 0, 1 oder 2 bedeutet,
und ihre Salze.

1-4C-Alkyl steht für geradkettige oder verzweigte Alkylreste mit 1 bis 4 Kohlenstoffatomen. Beispielsweise seien genannt der Butyl-, iso-Butyl-, sec.-Butyl-, tert.-Butyl-, Propyl-, Isopropyl-, Ethyl- und insbesondere der Methylrest.

1-4C-Alkoxy steht für ein Sauerstoffatom, an das einer der vorstehend genannten 1-4C-Alkylreste gebunden ist. Beispielsweise genannt seien der Methoxy-, der Ethoxy-, der Propoxy- und der Butoxyrest.

Als beispielhafte, durch R16 substituierte Phenylreste seien die Reste 4-Benzoylphenyl und 3-Hydroxyphenyl genannt.

2-4C-Alkyl steht für einen Alkylrest mit 2 bis 4 Kohlenstoffatomen. Beispielsweise seien der Ethyl-, der Propyl- und der Butylrest genannt.

1-4C-Alkoxycarbonyl steht für einen Rest, der neben der Carbonylgruppe einen der vorstehend genannten 1-4C-Alkoxyreste enthält. Beispielsweise seien der Methoxycarbonyl- und der Ethoxycarbonylrest genannt.

1-4C-Alkoxy-1-4C-alkoxy steht für einen der vorstehend genannten 1-4C-Alkoxyreste, der durch einen weiteren 1-4C-Alkoxyrest substituiert ist. Beispielsweise genannt seien die Reste 2-(Methoxy)ethoxy (-O-CH₂-CH₂-O-CH₃) und 2-(Ethoxy)ethoxy (-O-CH₂-CH₂-O-CH₂-CH₃).

Beispielhafte durch R11 substituierte 2-4C-Alkylreste sind 2-[2-(Methoxy)ethoxy)]ethyl und 2-[2-(Ethoxy)ethoxy]ethyl.

Beispielhafte durch R12 substituierte 1-4C-Alkylreste sind (Methoxycarbonyl)methyl, (Ethoxycarbonyl)methyl, (Propoxycarbonyl)methyl, 2-(Methoxycarbonyl)ethyl, 2-(Ethoxycarbonyl)ethyl, 2-(Propoxycarbonyl)ethyl, 3-(Methoxycarbonyl)propyl, Carboxymethyl, 2-Carboxyethyl, 3-Carboxypropyl, 4-Carboxybutyl, 2-Thienylmethyl, 2-(2-Thienyl)ethyl, 3-Thienylmethyl, 2-(3-Thienyl)ethyl, 3-(2-Thienyl)propyl, 3-(3-Thienyl)propyl, (3-Hydroxyphenyl)methyl, 2-(3-Hydroxyphenyl)ethyl, 3-(3-Hydroxyphenyl)propyl, 2-Pyridylmethyl, 3-Pyridylmethyl, 4-Pyridylmethyl, 2-(2-Pyridyl)ethyl, 3-(2-Pyridyl)propyl und 2-(1-Pyridyl)ethyl.

Als beispielhafte durch R15 substituierte 2-4C-Alkylreste seien genannt 2-(Phthalimidyl)ethyl, 3-(Phthalimidyl)propyl, 2-(Methylthio)ethyl, 2-(Ethylthio)ethyl, 2-(Propylthio)ethyl, 3-(Methylthio)propyl, 3-(Ethylthio)propyl, 2-(Methylsulfinyl)ethyl, 2-(Ethytsulfinyl)ethyl, 2-(Propylsulfinyl)ethyl, 2-(Methylsulfonyl)ethyl, 2-(Ethylsulfonyl)ethyl und 2-(Propylsulfonyl)ethyl.

Als beispielhafte Reste -SO₂-R6 seien genannt Isopropyl-, Methyl-, Propyl-, Ethyl- und Butylsulfonyl.

Beispielhafte Reste COO-R8 sind der (3-Hydroxyphenyl-1-)methoxycarbonyl-, 2-(Methylthio)ethoxycarbonyl-, 2-(Ethylthio)ethoxycarbonyl-, 2-(Methylsulfinyl)ethoxycarbonyl-, 2-(Ethylsulfinyl)ethoxycarbonyl-, 2-(Methylsulfonyl)ethoxycarbonyl-, 2-(Ethylsulfonyl)ethoxycarbonyl-, 2-(-Phthalimidyl)ethoxycarbonyl-, 2-[2-(Methoxy)ethoxy]ethoxycarbonyl- und der 2-[2-(Ethoxy)ethoxy]ethoxycarbonylrest.

Als Salze kommen für Verbindungen der Formel I - je nach Substitution - alle Säureadditionssalze oder alle Salze mit Basen in Betracht. Besonders erwähnt seien die pharmakologisch verträglichen Salze der in der Galenik üblicherweise verwendeten anorganischen und organischen Säuren und Basen. Als solche eignen sich einerseits wasserlösliche und wasserunlösliche Säureadditionssalze mit Säuren wie beispielsweise Salzsäure, Bromwasserstoffsäure, Phosphorsäure, Salpetersäure, Schwefelsäure, Essigsäure, Zitronensäure, D-Gluconsäure, Benzoesäure, 2-(4-Hydroxybenzoyl)-benzoesäure, Buttersäure, Sulfosalicylsäure, Maleinsäure, Laurinsäure, Äpfetsäure, Fumarsäure, Bemsteinsäure, Oxalsäure, Weinsäure, Embonsäure. Stearinsäure, Toluolsulfonsäure, Methansulfonsäure oder 3-Hydroxy-2-naphthoesäure, wobei die Säuren bei der Salzherstellung - je nachdem, ob es sich um eine ein- oder mehrbasige Säure handelt und je nachdem, welches Salz gewünscht wird - im äquimolaren oder einem davon abweichenden Mengenverhältnis eingesetzt werden.

Andererseits kommen auch Salze mit Basen in Betracht. Als Beispiele für Salze mit Basen seien Alkali- (Lithium-, Natrium-, Kalium-) oder Calcium-, Aluminium-, Magnesium-, Titan-, Ammonium-, Meglumin- oder Guanidiniumsalze erwähnt, wobei auch hier bei der Salzherstellung die Basen im äquimolaren oder einem davon abweichenden Mengenverhältnis eingesetzt werden.

Pharmakologisch unverträgliche Salze, die beispielsweise bei der Herstellung der erfindungsgemäßen Verbindungen im industriellen Maßstab als Verfahrensprodukte zunächst anfallen können, werden durch dem Fachmann bekannte Verfahren in pharmakologisch verträgliche Salze übergeführt.

Hervorzuhebende besonders bevorzugte Verbindungen der Formel I sind solche, worin
- R0: 1-4C-Alkyl bedeutet,
- R1: 1-4C-Alkyl bedeutet,
- R2: 1-4C-Alkyl bedeutet,
- R3: COO-R8 bedeutet,
- A: NH bedeutet,
- R8: durch R11 substituiertes 2-4C-Alkyl, durch R12 substituiertes 1-4C-Alkyl, -N=C(R13)R14 oder durch R15 substituiertes 2-4C-Alkyl bedeutet,
- R11: 1-4C-Alkoxy-1-4C-alkoxy bedeutet,
- R12: 1-4C-Alkoxycarbonyl, Carboxy, Thienyl, Pyridyl oder durch R16 substituiertes Phenyl bedeutet,
- R13: 1-4C-Alkyl und
- R14: 1-4C-Alkyl bedeuten,
- R15: -S(O)ₙ-R19 bedeutet, wobei
- R16: Hydroxy bedeutet,
- R19: 1-4C-Alkyl bedeutet,
- n: die Zahlen 0.1 oder 2 bedeutet,
und ihre Salze.

Eine Ausgestaltung der hervorzuhebenden besonders bevorzugten Verbindungen der Formel I sind solche, worin
- R0: 1-4C-Alkyl bedeutet,
- R1: 1-4C-Alkyl bedeutet,
- R2: 1-4C-Alkyl bedeutet,
- R3: COO-R8 bedeutet,
- A: NH bedeutet,
- R8: durch R11 substituiertes 2-4C-Alkyl oder durch R15 substituiertes 2-4C-Alkyl bedeutet,
- R11: 1-4C-Alkoxy-1-4C-alkoxy bedeutet,
- R15: -S(O)ₙ-R19 bedeutet, wobei
- R19: 1-4C-Alkyl bedeutet,
- n: die Zahlen 0,1 oder 2 bedeutet,
und ihre Salze.

Weiterer Gegenstand der Erfindung ist ein Verfahren zur Herstellung der Verbindungen der Formel I und ihrer Salze. Das Verfahren ist dadurch gekennzeichnet, daß man
a) Verbindungen der Formel II (siehe beigefügtes Formeiblatt), worin R0, R1, R2 und A die oben angegebenen Bedeutungen haben, mit Verbindungen der Formel R3-X, worin R3 die oben genannte Bedeutung besitzt und X eine geeignete Abgangsgruppe darstellt, umsetzt, oder daß man
b) Verbindungen der Formel III (siehe beigefügtes Formelblatt), worin R0, R1 und A die oben angegebenen Bedeutungen haben, mit Verbindungen der Formel IV (siehe beigefügtes Formelblatt), worin R2 und R3 die oben angegebenen Bedeutungen haben und X eine geeignete Abgangsgruppe darstellt umsetzt, oder daß man
c) Verbindungen der Formel I, worin R0, R1, R2 und A die oben angegebenen Bedeutungen haben und der Rest R3 eine Sulfid-Gruppe (-S-) enthält, oxidiert, oder daß man
d) Verbindungen der Formel I, worin R0, R1, R2 und A die oben angegebenen Bedeutungen haben und der Rest R3 eine Ether-Gruppe (-O-) enthält an der Ether-Gruppe zum entsprechenden terminalen Hydroxid (-OH) spaltet,
und daß man gewünschtenfalls anschließend die nach a), b), c) oder d) erhaltenen Verbindungen I in ihre Salze überführt, oder daß man gewünschtenfalls anschließend aus erhaltenen Salzen der Verbindungen I die Verbindungen I freisetzt.

Die Einführung des Restes R3 in die Verbindungen II nach Verfahrensvariante a) erfolgt auf eine dem Fachmann an sich vertraute Weise, beispielsweise so wie in den nachfolgenden Beispielen beschrieben. Beispielhafte erfindungsgemäße Verbindungen der Formel R3-X, worin X eine geeignete Abgangsgruppe bedeutet, sind entsprechende Sulfonsäurechloride, Chlorameisensaureester oder Säurechloride.

Welche Reaktionsbedingungen für die Durchführung des Verfahrens im einzelnen erforderlich sind, ist dem Fachmann aufgrund seines Fachwissens geläufig.

Die Umsetzung der Verbindungen III mit den Verbindungen IV nach Verfahrensvariante b) erfolgt ebenfalls auf eine dem Fachmann an sich vertraute Weise, beispielsweise unter analoger Anwendung solcher Verfahren, wie sie in den europäischen Patentanmeldungen EP-A-0 268 989 oder EP-A-0 308 917 beschrieben sind.

Eine geeignete Abgangsgruppe X in Verbindungen der Formel IV, ist beispielsweise ein Halogenatom (bevorzugt Chlor oder Brom) oder eine Methansulfonyloxygruppe. Die Umsetzung erfolgt vorteilhafterweise in Gegenwart einer Base (z.B. eines anorganischen Hydroxids, wie Natriumhydroxid, oder eines anorganischen Carbonates, wie Natriumcarbonat, oder einer organischen Stickstoffbase, wie Triethylamin, Pyridin, Kollidin oder 4-Dimethylaminopyridin), wobei durch Zusatz von Katalysatoren, wie Alkalijodid oder Tetrabutylammoniumbromid, die Reaktionsführung begünstigt werden kann.

Die Oxidation der Sulfide zu den Sulfoxiden bzw. Sulfonen analog Verfahrensvariante c) erfolgt unter den Bedingungen, wie sie dem Fachmann für die Oxidation von Sulfiden zu Sulfoxiden und Sulfonen geläufig sind [siehe hierzu z.B. J. Drabowicz und M. Mikolajczyk, Organic preparations and procedures int. 14(1-2), 45-89(1982) oder E. Block in S. Patai, The Chemistry of Functional Groups, Supplement E. Part 1, S. 539-608, John Wiley and Sons (Interscience Publication), 1980]. Als Oxidationsmittel kommen alle für die Oxidation von Sulfiden zu Sulfoxiden und Sulfonen üblicherweise verwendeten Reagenzien in Frage, insbesondere Peroxysäuren, wie z.B. Peroxyessigsäure, Trifluorperoxyessigsäure, 3,5-Dinitroperoxybenzoesäure, Peroxymaleinsäure, Magnesiummonoperoxiphthalat oder bevorzugt m-Chlorperoxybenzoesäure.

Die Reaktionstemperatur liegt (je nach Reaktivität des Oxidationsmittels und Verdünnungsgrad) zwischen -70°C und der Siedetemperatur des verwendeten Lösungsmittels, bevorzugt jedoch zwischen -30° und +20°C. Als vorteilhaft hat sich auch die Oxidation mit Halogenen bzw. mit Hypohalogeniten (z.B. mit wäßriger Natriumhypochloritlösung) erwiesen, die zweckmäßigerweise bei Temperaturen zwischen 0° und 50°C durchgeführt wird. Die Reaktion wird beispielsweise in inerten Lösungsmitteln, z.B. aromatischen oder chlorierten Kohlenwasserstoffen, wie Benzol, Toluol, Dichlormethan oder Chloroform, vorzugsweise in Estern oder Ethern, wie Essigsäureethylester, Essigsäureisopropylester oder Dioxan, oder in Alkoholen, vorzugsweise Isopropanol, durchgeführt.

Die erfindungsgemäßen Sulfoxide sind optisch aktive Verbindungen. Je nach Art der Substituenten können noch weitere Chiralitätszentren im Molekül sein. Die Erfindung umfaßt daher sowohl die Enantiomeren und Diastereomeren als auch ihre Mischungen und Racemate. Die Enantiomeren können in an sich bekannter Weise (beispielsweise durch Herstellung und Trennung entsprechender diastereoisomerer Verbindungen) separiert werden (siehe z.B. WO92/08716).

Die Etherspaltung bei Verbindungen der Formel I nach Variante d) erfolgt ebenfalls auf eine dem Fachmann bekannte Weise.

Die Isolierung und Reinigung der erfindungsgemäßen Substanzen erfolgt in an sich bekannter Weise z.B. derart, daß man das Lösungsmittel im Vakuum abdestilliert und den erhaltenen Rückstand aus einem geeigneten Lösungsmittel umkristallisiert oder einer der üblichen Reinigungsmethoden, wie beispielsweise der Säulenchromatographie an geeignetem Trägermaterial, unterwirft.

Säureadditionssalze erhält man durch Auflösen der freien Base in einem geeigneten Lösungsmittel, z.B. in Wasser, in einem chlorierten Kohlenwasserstoff, wie Methylenchlorid oder Chloroform, einem niederen aliphatischen Alkohol (Ethanol, Isopropanol), einem Keton, wie Aceton, oder einem Ether, wie THF oder Diisopropylether, das die gewünschte Säure enthält, oder dem die gewünschte Säure anschließend zugegeben wird.

Die Salze werden durch Filtrieren, Umfällen, Ausfällen mit einem Nichtlösungsmittel für das Anlagerungssalz oder durch Verdampfen des Lösungsmittels gewonnen. Erhaltene Salze können durch Alkalisierung, z.B. mit wäßriger Ammoniaklösung, in die freien Basen umgewandelt werden, welche wiederum in Säureadditionssalze übergeführt werden können. Auf diese Weise lassen sich pharmakologisch nicht verträgliche Säureadditionssalze in pharmakologisch verträgliche Säureadditionssalze umwandeln.

Die Verbindungen der Formel R3-X, worin X eine geeignete Abgangsgruppe, bevorzugt Chlor bedeutet, sind entweder bekannt, oder können auf bekannte Weise hergestellt werden. Beispielsweise können benötigte Chlorameisensäureester aus den entsprechenden Alkoholen durch Umsetzung mit Phosgen erhalten werden.

Die Ausgangsverbindungen III sind aus der europäischen Patentanmeldung EP-A-0299 470 bekannt oder können auf analoge Weise hergestellt werden.

Die Ausgangsverbindungen IV können auf analoge Weise wie in der europäischen Patentanmeldung EP-A-0 308 917 beschrieben hergestellt werden. Alternativ kann die Herstellung auch ausgehend von Verbindungen der Formel IV, worin R3 die Bedeutung Wasserstoff hat, erfolgen. Dazu werden diese Verbindungen zuerst in das entsprechende Isocyanat übergeführt und durch anschließende Reaktion mit einem entsprechenden Amin, Oxim oder Alkohol zur gewünschten Verbindung der Formel IV umgesetzt.

Die folgenden Beispiele dienen der näheren Erläuterung zur Herstellung der erfindungsgemäßen Verbindungen. Insbesondere dienen die Beispiele auch dazu, die Umsetzungen gemäß den Verfahrensvarianten a), b), c) und d), sowie die Herstellung ausgewählter Ausgangsverbindungen exemplarisch zu beschreiben. Ebenso können weitere Verbindungen der Formel I sowie weitere Ausgangsverbindungen, deren Herstellung nicht explizit beschrieben ist, in analoger oder in einer dem Fachmann an sich vertrauten Weise unter Anwendung üblicher Verfahrenstechniken hergestellt werden. Die Abkürzung RT steht für Raumtemperatur, h steht für Stunde(n), min für Minute(n), Schmp. für Schmelzpunkt, Zers. für Zersetzung und Sdp. für Siedepunkt.

Die in den Beispielen genannten Verbindungen und ihre Salze sind bevorzugter Gegenstand der Erfindung.

### Beispiele

### Ausgangsverbindungen

### A1. (2-Chlormethyl-3-methyl-phenyl)carbamidsäure-2-(N-phthalimido-2-ethyl)-ester

Eine Suspension von 2-Chlormethyl-1-isocyanato-3-methylbenzol (1 g) und N-(2-Hydroxyethyl)-phthalimid (1,07 g) in Petrolether (Sdp. 100-140°C) (100 ml) wird 8 h am Rückfluß erhitzt und anschließend noch 16 h bei RT nachgerührt Der Niederschlag wird abfiltriert und mit wenig Dichlormethan und Diethylether nachgewaschen. Man erhält 1,06 g (52 %) der Titelverbindung als Feststoff vom Schmp. 176-178°C.

### A2. 8-(2-tert.-Butoxycarbonylamino-6-methyl-benzylamino)-2,3-dimethyl-imidazo[1,2-a]pyridin

Zu einer Lösung von 8-Amino-2,3-dimethylimidazo[1,2-a]pyridin (4,8 g) und 2-tert.-Butoxycarbonylamino-6-methylbenzylchlorid (9,2 g) in Aceton (250 ml) gibt man bei RT 5,5 g Natriumjodid und 8,0 g Natriumcarbonat und erhitzt anschließend für 6 h unter Rückfluß zum Sieden. Nach Abkühlen der Lösung auf RT und Einengen wird der Rückstand in einem Gemisch aus 200 ml Ethylacetat und 200 ml Wasser gelöst und die organische Phase wird abgetrennt. Nach drei weiteren Extraktionen mit jeweils 100 ml Ethylacetat werden die vereinigten organischen Phasen Ober Magnesiumsulfat getrocknet und anschließend eingeengt. Die Titelverbindung kristallisiert als schwach gelber Feststoff. Nach chromatografischer Reinigung an Kieselgel (Fließmittel: Toluol/Dioxan = 20:1) und Umkristallisation aus Diisopropylether erhält man 7,1 g (62 %) der Titelverbindung vom Schmp. 149-152°C.

### A3. 8-(2-Amino-6-methyl-benzylamino)-2,3-dimethyl-imidazo[1,2-a]pyridin

### Methode A:

Eine Lösung von 8-(6-Methyl-2-nitrobenzylamino)-2,3-dimethylimidazo[1,2-a]pyridin (61 g) in Methanol (5,5 l) wird in Gegenwart von 15 g Palladium auf Aktivkohle (5 %) als Katalysator bei RT und unter Atmosphärendruck für 1,5 h hydriert. Nach Abfiltrieren des Katalysators und Einengen wird der Rückstand in siedendem Ethylacetat gelöst (2,7 l). Nach dem Abkühlen auf R-T werden 51 g (82 %) der Titelverbindung vom Schmp. 206-208°C isoliert.

### Methode B:

6.7 g 8-(2-tert.-Butoxycarbonylamino-6-methylbenzylamino)-2,3-dimethylimidazo[1,2-a]pyridin werden bei 25-30°C portionsweise zu einer Mischung aus Trifluoressigsäure (30 ml) und Anisol (3 ml) zugefügt. Nach 30-minütigem Rühren bei RT wird die Lösung in 100 ml Eiswasser gegossen und anschließend mit 75 ml 6 N Natronlauge versetzt Der Niederschlag wird abfiltriert und an Kieselgel chromatografisch gereinigt (Lösungsmittel: Toluol/Dioxan = 8:1). Nach dem Umkristallisieren aus Ethylacetat erhält man 3,1 g (62 %) der Titelverbindung vom Schmp. 206-208°C.

### B1. (2-Hydroxymethyl-3-methyl-phenyl)carbamidsäure(methoxycarbonylmethyl)ester

Ausgehend von Methoxycarbonylmethyl-chloroformat (11,7 g), 2-Amino-6-methyl-benzylalkohol (10,5 g) und Pyridin (6,2 ml) in Dichlormethan (230 ml) erhält man nach der in Beispiel C1 angegebenen Arbeitsweise, nach Chromatographie an Kieselgel (Fließmittel: Essigester/Petrolether 50/70 = 1:1) und Kristallisation aus Diisopropylether 6,8 g (20 %) der Titelverbindung, Schmp. 70-74°C.

### B2. (2-Chlormethyl-3-methyl-phenyl)carbamidsäure(methoxycarbonylmethyl)ester

(2-Hydroxymethyl-3-methyl-phenyl)carbamidsäure(methoxycarbonylmethyl)ester (6,8 g) wird in Dichlormethan (60 ml) vorgelegt und tropfenweise mit Thionylchlorid (3,4 ml) versetzt. Danach wird noch 1 h bei RT gerührt. Dann wird das Lösungsmittel abdestilliert und der Rückstand 2mal mit Dichlormethan (je 50 ml) versetzt und am Rotationsverdampfer jeweils wieder eingeengt. Man erhält 7 g (96 %) der Titelverbindung vom Schmp. 114-116°C.

### C1. 2-Chlormethyl-1-Isocyanato-3-methylbenzol

2-Amino-6-methyl-benzylalkohol (10,0 g) wird in abs. Toluol (200 ml) bei 50°C gelöst. Bei dieser Temperatur tropft man 45 ml ca. 3,5 M etherische Salzsäure während 0,5 h zu. Die entstandene Suspension wird 2 h bei RT nachgerührt. Dann werden 5,3 ml Thionylchlorid bei dieser Temperatur zugetropft (Gasentwicklung). Die Suspension wird noch 16 h gerührt und anschließend am Rotationsverdampfer (Badtemperatur 40°C) eingeengt. Der Rückstand wird 3mal in jeweils 200 ml Toluol aufgenommen und immer wieder eingeengt. Der kristalline Rückstand wird in 400 ml wasserfreiem Toluol suspendiert und im Ölbad auf 50°C erwärmt. Dann wird Trichlormethylchloroformat (11,3 ml) zugetropft. Nach vollständiger Zugabe wird 5 h am Rückfluß erhitzt, bis die Gasentwicklung beendet ist. Anschließend wird das Toluol im Vakuum abgezogen und Rückstand im Hochvakuum destilliert. Man erhält 6,55 g (49 %) der Titelverbindung als farblose Flüssigkeit vom Kp (0,25 mbar) 92-93°C.

### C2. O-(2-Chlormethyl-3-methyl-phenylaminocarbonyl)-acetonoxim

Eine Lösung von 2-Chlormethyl-1-isocyanato-3-methylbenzol (2 g) und Acetonoxim (0,85 g) in n-Hexan (20 ml) wird 16 h bei RT gerührt, dabei fällt ein Niederschlag aus. Nach Filtration und Nachwaschen mit n-Hexan und Diethylether erhält man 2,4 g (86 %) der Titelverbindung als Feststoff vom Schmp. 76-78°C.

### C3. (2-Chlormethyl-3-methyl-phenyl)carbamidsäure-2-(2-methoxy-ethoxy)ethylester

Eine Lösung von 2-Chlormethyl-1-isocyanato-3-methylbenzol (2 g) und Diethylenglykol-monomethylether (1,32 g) in n-Hexan (50 ml) wird 5 h am Rückfluß erhitzt. Nach Abkühlen auf RT erhält man eine Suspension. Durch Filtration und Nachwaschen mit n-Hexan und Diethylether isoliert man 1,3 g (39 %) der Titelverbindung als Feststoff vom Schmp. 78-83°C.

### C4. (2-Chlormethyl-3-methyl-phenyl)carbamidsäure-(2-thienylmethyl)ester

2-Chlormethyl-1-isocyanato-3-methylbenzol (2,5 g) und Thiophenmethanol (1,57 g) werden vereint und bei RT gerührt. Bei einsetzender Kristallisation gibt man Petrolether 50/70 (20 ml) zu und rührt die Suspension 16 h bei RT. Nach Filtration und Nachwaschen mit Diethylether erhält man 2,57 g (63 %) der Titelverbindung als Feststoff vom Schmp. 111-114°C.

### C5. (2-Chlormethyl-3-methyl-phenyl)carbamidsäure-(2-pyridinylmethyl)ester

Eine Lösung von 2-Chlormethyl-1-isocyanato-3-methylbenzol (2,5 g) und 2-Hydroxymethylpyridin (1,5 g) in Dichlormethan (25 ml) wird 16 h bei RT gerührt. Durch Aufkonzentration am Rotationsverdampfer (Badtemperatur 22°C) und anschließender Chromatographie an Kieselgel (Fließmittel: Dichlormethan/Methanol 13:1) erhält man eine Lösung der Titelverbindung, welche am Rotationsverdampfer (Badtemperatur 22°C) aufkonzentriert wird, (nicht Kristallisieren, sonst Zersetzung). Das erhaltene Rohprodukt der Titelverbindung wird sofort weiter umgesetzt.

### C6. (2-Chlormethyl-3-methyl-phenyl)carbamidsäure-(3-hydroxybenzyl)-ester

Eine Mischung von 2-Chlormethyl-1-isocyanato-3-methylbenzol (2 g) und 3-Hydroxybenzylalkohol (1,6 g) wird 1 h im Ölbad auf 75°C erwärmt und anschließend durch Chromatographie an Kieselgel (Fließmittel: Dichlormethan/Methanol 19:1) gereinigt. Man erhält 1 g (30 %) der Titelverbindung als Rohprodukt in Form eines amorphen Feststoffs.

### C7. (2-Chlormethyl-3-methyl-phenyl)-carbamidsäure-(2-N-phthalimidoethyl)-ester

Eine Suspension von 2-Chlormethyl-1-isocyanato-3-methylbenzol (1,0 g) und 2-Hydroxyethyl-N--phthalimid (1,07 g) in Petrolether 100/140 (100 ml) wird 8 h am Rückfluß erhitzt. Danach wird noch 16 h bei RT nachgerührt. Der Niederschlag wird abfiltriert mit wenig Dichlormethan und mit Diethylether nachgewaschen und im Hochvakuum getrocknet. 1,06 g (52%) der Titelverbindung werden als hellbeiger Feststoff isoliert. Schmp. 176-178°C.

### Endprodukte

### 1. Ethansulfonsäure-[2-(2,3-dimethyl-imidazo[1,2-a]pyridin-8yl-amino-methyl)-3-methyl--phenyl]-amid

Eine Lösung von 8-(2-Amino-6-methyl-benzylamino)-2,3-dimethyl-imidazo[1,2-a]pyridin (1,86 g), Pyridin (1,1 ml) und Ethansulfonsäure-chlorid (1,18 ml) in Dichlormethan (40 ml) wird 48 h bei RT gerührt. Danach wird die Reaktionmischung mit Dichlormethan (60 ml) verdünnt, auf Wasser (100 ml) gegeben und extrahiert: Die organische Phase wird mit Natriumhydrogencarbonat-Lösung (60 ml) und mit Wasser (60 ml) gewaschen, über Natriumsulfat getrocknet und eingeengt. Durch Kristallisation des Rückstands aus Aceton und anschließende Filtration erhält man 0,8 g (32 %) der Titelverbindung vom Schmp. 240-242°C.

### 2. n-Propansulfonsäure-[2-(2,3-dimethyl-imidazo[1,2-a]pyridin-8ylamino-methyl)-3-methyl-phenyl]-amid

Eine Lösung von 8-(2-Amino-6-methyl-benzylamino)-2,3-dimethyl-imidazo-[1,2-a]pyridin (2,5 g), Pyridin (1,4 ml) und n-Propansulfonsäurechlorid (2 ml) in Dichlormethan (50 ml) wird 5 Tage bei RT gerührt. Danach wird die Reaktionsmischung mit Dichlormethan (50 ml) verdünnt, auf Wasser gegeben und extrahiert. Die organische Phase wird mit Natriumhydrogencarbonat-Lösung (50 ml) und mit Wasser (50 ml)gewaschen, über Natriumsulfat getrocknet und eingeengt. Durch Kristallisation des Rückstands aus Aceton und anschließende Filtration erhält man 1,8 g (52 %) der Titelverbindung vom Schmp. 245-248°C.

### 3. 8-{2-[(2-Methoxyethoxy-2-ethoxy)carbonylamino]-6-methyl-benzylamino}-2,3-dimethyl-imidazo[1,2-a]pyridin-Hydrochlorid

Eine Suspension von 8-Amino-2,3-dimethyl-imidazo[1,2-a]pyridin (0,48 g), (2-Chlormethyl-3-methyl-phenyl)carbamidsäure-2-(2-methoxyethoxy)ethylester (0,9 g), Natriumcarbonat (0,8 g) und Natriumjodid (0,15 g) in Aceton (20 ml) wird 16 h bei RT gerührt. Danach wird der Niederschlag abfiltriert, mit Aceton nachgewaschen und das Filtrat am Rotationsverdampfer eingeengt. Der Rückstand wird in Natriumhydrogencarbonat-Lösung (50 ml) aufgenommen und mit Dichlormethan (3 x 50 ml) extrahiert. Die vereinigten organische Extrakte werden mit Wasser (100 ml) gewaschen, über Natriumsulfat getrocknet und eingeengt. Der Rückstand wird durch zweimalige Chromatographie an Kieselgel (Fließmittel A: Dichlormethan/Methanol 13:1, Fließmittel B: Essigester) gereinigt. Die Hauptfraktion wird mit etherischer Salzsäure versetzt und eingeengt. Man erhält 0.7 g (50 %) der Titelverbindung als amorphen Feststoff, der bei 70-80°C sintert.

### 4. 8-{2-[O-(Carbamoyl)acetonoxim]-6-methyl-benzylamino}-2,3-dimethyl-imidazo[1,2-a]pyridin

Eine Suspension von 8-Amino-2,3-dimethyl-imidazo[1,2-a]pyridin (0,63 g), O-(2-Chlormethyl-3-methyl-phenylcarbonyl)acetonoxim (1 g), Natriumcarbonat (1,04 g) und Natriumjodid (0,2 g) in Aceton (20 ml) wird 16 h bei RT gerührt. Der Niederschlag wird abfiltriert, mit Aceton nachgewaschen und das Filtrat am Rotationsverdampfer eingeengt. Der Rückstand wird in Natriumhydrogencarbonat-Lösung (50 ml) aufgenommen und mit Dichlormethan (3 x 50 ml) extrahiert. Die vereinigten organische Extrakte werden mit Wasser (50 ml) gewaschen, über Natriumsulfat getrocknet und eingeengt. Der Rückstand wird durch Chromatographie an Kieselgel (Fließmittel: Dichlormethan/Methanol 13:1) gereinigt. Nach Einengen der Hauptfraktion und Kristallisation aus Diethylether erhält man 0,92 g (62 %) der Titelverbindung vom Schmp. 138°C.

### 5. 8-{2-[(2-Pyridinyl-methoxy)carbonylamino]-6-methyl-benzylamino}-2,3-dimethyl-imidazo[1,2-a]pyridin

Eine Suspension von 8-Amino-2,3-dimethyl-imidazo[1,2-a]pyridin (0,58 g), (2-Chlormethyl-3-methyl-phenyl)-carbamidsäure-(2-pyridinylmethyl)ester (1.04 g), Natriumcarbonat (0,95 g) und Natriumjodid (0,2 g) in Aceton (25 ml) wird 16 h bei RT gerührt. Der Niederschlag wird abfiltriert, mit Aceton nachgewaschen und das Filtrat am Rotationsverdampfer eingeengt. Der Rückstand wird in Natriumhydrogencarbonat-Lösung (40 ml) aufgenommen und mit Dichlormethan (3 x 40 ml) extrahiert. Die vereinigten organische Extrakte werden mit Wasser (70 ml) gewaschen, über Natriumsulfat getrocknet und eingeengt. Der Rückstand wird durch Chromatographie an Kieselgel (Fließmittel: Essigester) gereinigt. Nach Einengen der Hauptfraktion und Kristallisation aus Aceton erhält man 0,4 g (27 %) der Titelverbindung vom Schmp. 163-165°C.

### 6. 8-{2-[(2-Thienyl-methoxy)carbonylamino]-6-methyl-benzylamino}-2,3-dimethyl-imidazo[1,2-a]pyridin

Eine Suspension von 8-Amino-2,3-dimethyl-imidazo[1,2-a]pyridin (0,68 g), (2-Chlormethyl-3-methyl-phenyl)carbamidsäure-2-(2-thienyl-methyl)ester (1,25 g), Natriumcarbonat (1,12 g) und Natriumjodid (0,16 g) in Aceton (40 ml) wird 16 h bei RT gerührt. Der Niederschlag wird abfiltriert, mit Aceton nachgewaschen und das Filtrat am Rotationsverdampfer eingeengt. Der Rückstand wird in Natriumhydrogencarbonat-Lösung aufgenommen und mit Dichlormethan (3 x 50 ml) extrahiert. Die vereinigten organischen Extrakte werden mit Wasser (50 ml) gewaschen, über Natriumsulfat getrocknet und eingeengt. Der Rückstand wird durch Chromatographie an Kieselgel (Fließmittel: Essigester) gereinigt. Nach Einengen der Hauptfraktion und Kristallisation aus Essigester/Diisopropylether erhält man 0,61 g (34 %) der Titelverbindung vom Schmp. 146-147°C.

### 7. 8-{2-[(3-Hydroxybenzyloxy)carbonylamino]-6-methyl-benzylamino}-2,3-dimethyl-imidazo[1,2-a]pyridin

Eine Suspension von 8-Amino-2,3-dimethyl-imidazo[1,2-a]pyridin (0,63 g), (2-Chlormethyl-3-methyl-phenyl)carbamidsäure-3-hydroxybenzylester (1,2 g), Natriumcarbonat (1 g) und Natriumjodid (0,14 g) in Aceton (20 ml) wird 16 h bei RT gerührt. Der Niederschlag wird abfiltriert, mit Aceton nachgewaschen und eingeengt. Der Rückstand wird in Natriumhydrogencarbonat-Lösung (40 ml) aufgenommen und mit Dichlormethan (3 x 40 ml) extrahiert. Die vereinigten organischen Extrakte werden mit Wasser gewaschen, über Natriumsulfat getrocknet und eingeengt. Der Rückstand wird durch Flash-Chromatographie an Kieselgel (Fließmittel: Dichlormethan/ Methanol 13:1) gereinigt. Nach Einengen der Hauptfraktion und Kristallisation aus Aceton erhält man 0,4 g (22 %) der Titelverbindung vom Schmp. 190-193°C.

### 8. 8-{2-[(Hydroxycarbonylmethyloxy)carbonylamino]-6-methyl-benzyl-amino}-2,3-dimethyl-imidazo[1,2-a]pyridin-Hydrochlorid

### A) 8-{2-[(Methoxycarbonyl-methoxycarbonylamino]-6-methyl-benzyl-amino}-2,3-dimethyl-imidazo[1,2-a]pyridin

Eine Suspension von 8-Amino-2,3-dimethyl-imidazo[1,2-a]pyridin (2,05 g), (2-Chlormethyl-3-methyl-phenyl)-carbamidsäure-(methoxycarbonyl-methyl)ester (3,45 g), Natriumcarbonat (3,42 g) und Natriumjodid (0,59 g) in Aceton (60 ml) wird 20 h bei RT gerührt. Der Niederschlag wird abfiltriert, mit Aceton nachgewaschen und das Filtrat am Rotationsverdampfer eingeengt. Der Rückstand wird in Natriumhydrogencarbonat-Lösung aufgenommen und mit Dichlormethan (3 x 70 ml) extrahiert. Die vereinigten organischen Extrakte werden mit Wasser (100 ml) gewaschen, über Natriumsulfat getrocknet und eingeengt. Der Rückstand wird durch Chromatographie an Kieselgel (Fließmittel: Dichlormethan/ Methanol 13:1) gereinigt. Nach Einengen der Hauptfraktion erhält man 1 g eines schaumigen Produkts. Dieses Rohprodukt ist ein ca. 1:1 Substanzgemisch aus 3-[2-(2,3-Dimethyl-imidazo[1,2-a]pyridin-8-ylamino-methyl)-3-methylphenyl]-oxazolidin-2,4-dione und der Titelverbindung A, (Fließmittel zur Qualitätsüberprüfung: Dichlormethan/Methanol/Ammoniak = 13:1:0,5). Dieses Rohprodukt wurde direkt weiter umgesetzt.

### B) 8-{2-[(Hydroxycarbonylmethyloxy)carbonylamino]-6-methyl-benzyl-amino}-2,3-dimethyl-imidazo[1,2-a]pyridin-Hydrochlorid

Das ca. 50 %ige Rohprodukt aus A) (0,9 g) wird in Ethanol (10 ml) gelöst, mit einer Lösung aus Kaliumhydroxyd (0,15 g) in Ethanol (10 ml) versetzt und 21 h bei RT gerührt. Danach wird die Reaktionslösung eingeengt. Der Rückstand wird in 1 N Salzsäure (25 ml) aufgenommen und mit Dichlormethan (7 x 20 ml) extrahiert. Die vereinigten organischen Extrakte werden eingeengt und an Kieselgel chromatographiert (Fließmittel: Dichlormethan/Methanol 13:1 als Gradient bis 1:1). Die Fraktion mit Rf=0,05-0,1 (Fließmittel: Dichlormethan/Methanol = 13:1) wird eingeengt und im Hochvakuum bei 60°C 16 h getrocknet. Man erhält 0,2 g (21 %) der Titelverbindung vom Schmp. 114°C (sintert).

### 9. 2,3-Dimethyl-8-{2-[(2-methylsulfanylethoxy)carbonylamino]-6-methyl-benzylamino}imidazo[1,2-a]pyridin hemifumarat

Eine 20 %ige Lösung von Phosgen in Toluol (2,8 ml) wird mit wasserfreiem Dichlormethan (15 ml) verdünnt und auf 0°C gekühlt. Dann wird eine Lösung von 2-Methylsulfanylethanol (0,46 ml) und N-Methylmorpholin (0,59 ml) in wasserfreiem Dichlormethan (10 ml) zugetropft. Die Mischung wird noch 15 min bei 0°C gehalten, anschließend auf RT erwärmt und weitere 30 min gerührt. Anschließend wird zu der Lösung eine Suspension von 8-(2-Amino-6-methylbenzylamino)-2,3-dimethyl-imidazo[1,2-a]-pyridin (1,0 g) und Triethylamin (0,49 ml) in wasserfreiem Dichlormethan (30 ml) zugetropft und noch 1 h bei RT gerührt. Danach wird wässerige Natriumbicarbonatlösung (70 ml) zugegeben und noch 30 min gerührt. Die organische Phase wird abgetrennt und die wässerige Phase mit Dichlormethan (3 x 50 ml) extrahiert. Die vereinigten organischen Phasen werden mit Wasser (50 ml) gewaschen, über Magnesiumsulfat getrocknet und eingeengt. Der Rückstand wird in einer heißen Lösung von Fumarsäure (400 mg) in Aceton (30 ml) aufgenommen und auf 4°C abgekühlt. Der Niederschlag wird abfiltriert und noch aus wenig heißem Aceton ausgerührt. Nach Trocknung im Hochvakuum werden 0,65 g (47 %) der Titelverbindung als hellbeiges Kristallisat isoliert. Schmp. 174-176°C.

### 10. 2,3-Dimethyl-8-{2-[(2-methylsulfinylethoxy)carbonylamino]-6-methyl-benzylamino}imidazo[1,2-a]pyridin hemifumarat

Eine Lösung von 2,3-Dimethyl-8-{2-[(2-methylsulfanylethoxy)-carbonylamino]-6-methyl-benzylamino}-imidazo[1,2-a]pyridin (1,0 g) in wasserfreiem Dichlormethan (25 ml) wird auf 0°C gekühlt und portionsweise mit m-Chlorperoxibenzoesäure (800 mg) versetzt und dann noch 15 min bei 0°C gerührt. Anschließend wird wässerige Natriumbicarbonatlösung (25 ml) zugegeben, noch 10 min gerührt, die organische Phase abgetrennt und die wässerige Phase mit Dichlormethan (3 x 20 ml) extrahiert. Die vereinigten organischen Phasen werden mit Wasser (50 ml) gewaschen, über Magnesiumsulfat getrocknet und eingeengt. Zur Abtrennung des Sulfons wird der Rückstand an Kieselgel (Fließmittel: Essigester/Methanol/Ammoniak = 19:1:0,1) chromatographiert Die Fraktionen mit Rf = 0,15 werden gesammelt und eingeengt. Der Rückstand wird in einer heißen Lösung von Fumarsäure (150 mg) in Aceton (20 ml) aufgenommen und anschließend auf 4°C abgekühlt. Nach Filtration und Trocknung im Hochvakuum isoliert man 0,54 g (46 %) der Titelverbindung als hellbeiges Kristallisat. Schmp. 137-138°C.

### 11. 2,3-Dimethyl-8-{2-[(2-methylsulfonylethoxy)carbonylamino]-6-methyl-benzylamino}imidazo[1,2-a]pyridin

Eine Lösung von 2,3-Dimethyl-8-{2-[(2-methylsulfanylethoxy)-carbonylamino]-6-methyl-benzylamino}-imidazo[1,2-a]pyridin (500 mg) in wasserfreiem Dichlormethan (10 ml) wird auf 0°C gekühlt und portionsweise mit m-Chlorperoxibenzoesäure (0,87 g) versetzt und dann noch 45 min bei 0°C gerührt Anschließend wird wässerige Natriumbicarbonatlösung (15 ml) zugegeben, noch 10 min gerührt, die organische Phase abgetrennt und die wässerige Phase mit Dichlormethan (3 x 10 ml) extrahiert. Die vereinigten organischen Phasen werden mit Wasser (25 ml) gewaschen, über Magnesiumsulfat getrocknet und eingeengt. Zur Abtrennung von Nebenprodukten wird der Rückstand an Kieselgel (Fließmittel: Essigester/Methanol = 10:1) chromatographiert. Die Fraktionen mit Rf = 0,3 werden gesammelt und eingeengt. Der Rückstand wird aus Essigester/Diisopropylether kristallisiert. Nach Filtration und Trocknung im Hochvakuum isoliert man 250 mg (22 %) der Titelverbindung als hellbeiges Kristallisat. Schmp. 161-162°C.

### Gewerbliche Anwendbarkeit

Die Verbindungen der Formel I und ihre Salze besitzen wertvolle pharmakologische Eigenschaften, die sie gewerblich verwertbar machen. Sie weisen insbesondere eine ausgeprägte Magensäuresekretionshemmung und eine ausgezeichnete Magen- und Darmschutzwirkung bei Warmblütern auf. Hierbei zeichnen sich die erfindungsgemäßen Verbindungen durch eine hohe Wirkungsselektivität, eine vergleichsweise lange Wirkungsdauer, eine gute enterale Wirksamkeit, das Fehlen wesentlicher Nebenwirkungen und eine große therapeutische Breite aus.

Unter "Magen- und Darmschutz" wird in diesem Zusammenhang die Verhütung und Behandlung gastrointestinaler Krankheiten, insbesondere gastrointestinaler entzündlicher Krankheiten und Läsionen (wie z.B. Ulcus ventriculi, Ulcus duodeni, Gastritis, hyperazider oder medikamentös bedingter Reizmagen) verstanden, die beispielsweise durch Mikroorganismen (z.B. Helicobacter pylori), Bakterientoxine, Medikamente (z.B. bestimmte Antiphlogistika und Antirheumatika), Chemikalien (z.B. Ethanol), Magensäure oder Streßsituationen verursacht werden können. Die erfindungsgemäßen Verbindungen besitzen hierbei auch eine Eigenwirkung gegen den Keim Helicobacter pylori.

In ihren ausgezeichneten Eigenschaften erweisen sich die erfindungsgemäßen Verbindungen an verschiedenen Modellen, in denen die antiutcerogenen und die antisekretorischen Eigenschaften bestimmt werden, überraschenderweise den aus dem Stand der Technik bekannten Verbindungen deutlich überlegen. Aufgrund dieser Eigenschaften sind die Verbindungen der Formel I und ihre pharmakologisch verträglichen Salze für den Einsatz in der Human- und Veterinärmedizin hervorragend geeignet, wobei sie insbesondere zur Behandlung und/oder Prophylaxe von Erkrankungen des Magens und/oder Darms verwendet werden.

Ein weiterer Gegenstand der Erfindung sind daher die erfindungsgemäßen Verbindungen zur Anwendung bei der Behandlung und/oder Prophylaxe der vorstehend genannten Krankheiten. Ebenso umfaßt die Erfindung die Verwendung der erfindungsgemäßen Verbindungen zur Herstellung von Arzneimitteln, die zur Behandlung und/oder Prophylaxe der vorstehend genannten Krankheiten eingesetzt werden.

Weiterhin umfaßt die Erfindung die Verwendung der erfindungsgemäßen Verbindungen zur Behandlung und/oder Prophylaxe der vorstehend genannten Krankheiten.

Ein weiterer Gegenstand der Erfindung sind Arzneimittel, die ein oder mehrere Verbindungen der Formel I und/oder ihre pharmakologisch verträglichen Salze enthalten.

Die Arzneimittel werden nach an sich bekannten, dem Fachmann geläufigen Verfahren hergestellt. Als Arzneimittel werden die erfindungsgemäßen pharmakologisch wirksamen Verbindungen (= Wirkstoffe) entweder als solche, oder vorzugsweise in Kombination mit geeigneten pharmazeutischen Hilfs- oder Trägerstoffen in Form von Tabletten, Dragees, Kapseln, Suppositorien, Pflastern (z.B. als TTS), Emulsionen, Suspensionen oder Lösungen eingesetzt, wobei der Wirkstoffgehalt vorteilhafterweise zwischen 0,1 und 95 % beträgt und wobei durch die entsprechende Wahl der Hilfs- und Trägerstoffe eine auf den Wirkstoff und/oder auf den gewünschten Wirkungseintritt genau angepaßte galenische Darreichungsform (z.B. eine Retardform oder eine magensaftresistente Form) erzielt werden kann.

Welche Hilfs- bzw. Trägerstoffe für die gewünschten Arzneimittelformulierungen geeignet sind, ist dem Fachmann aufgrund seines Fachwissens geläufig. Neben Lösemitteln, Gelbildnern, Suppositoriengrundlagen, Tablettenhilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler, Farbstoffe oder insbesondere Permeationspromotoren und Komplexbildner (z.B. Cyclodextrine) verwendet werden.

Die Wirkstoffe können oral, parenteral oder percutan appliziert werden.

Im allgemeinen hat es sich in der Humanmedizin als vorteilhaft erwiesen, den oder die Wirkstoffe bei oraler Gabe in einer Tagesdosis von etwa 0,01 bis etwa 20, vorzugsweise 0,05 bis 5, insbesondere 0,1 bis 1,5 mg/kg Körpergewicht, gegebenenfalls in Form mehrerer, vorzugsweise 1 bis 4 Einzefgaben zur Erzielung des gewünschten Ergebnisses zu verabreichen. Bei einer parenteralen Behandlung können ähnliche bzw. (insbesondere bei der intravenösen Verabreichung der Wirkstoffe) in der Regel niedrigere Dosierungen zur Anwendung kommen. Die Festlegung der jeweils erforderlichen optimalen Dosierung und Applikationsart der Wirkstoffe kann durch jeden Fachmann aufgrund seines Fachwissens leicht erfolgen.

Sollen die erfindungsgemäßen Verbindungen und/oder Salze zur Behandlung der oben genannten Krankheiten eingesetzt werden, so können die pharmazeutischen Zubereitungen auch einen oder mehrere pharmakologisch aktive Bestandteile anderer Arzneimittelgruppen, wie Antacida, beispielsweise Aluminiumhydroxyd, Magnesiumaluminat; Tranquilizer, wie Benzodiazepine, beispielsweise Diazepam; Spasmolytika, wie z.B. Bietamiverin, Camylofin, Anticholinergica, wie z.B. Oxyphencyclimin, Phencarbamid; Lokalanaesthetika, wie z.B. Tetracain, Procain; gegebenenfalls auch Fermente, Vitamine oder Aminosäuren enthalten.

Hervorzuheben ist in diesem Zusammenhang insbesondere die Kombination der erfindungsgemäßen Verbindungen mit Pharmaka, die die Säuresekretion hemmen, wie beispielsweise H₂-Blockern (z.B. Cimetidin, Ranitidin). H⁺/K⁺-ATPase-Hemmstoffen (z.B. Omeprazol, Pantoprazol), oder ferner mit sogenannten peripheren Anticholinergika (z.B. Pirenzepin, Telenzepin) sowie mit Gastrin-Antagonisten mit dem Ziel, die Hauptwirkung in additivem oder überadditivem Sinn zu verstärken und/oder die Nebenwirkungen zu eliminieren oder zu verringern, oder ferner die Kombination mit antibakteriell wirksamen Substanzen (wie z.B. Cephalosporinen, Tetracyclinen, Nalidixinsäure, Penicillinen oder auch Wismutsalzen) zur Bekämpfung von Helicobacter pylori.

### Pharmakologie

Die ausgezeichnete Magenschutzwirkung und die magensäuresekretionshemmende Wirkung der erfindungsgemäßen Verbindungen kann in Untersuchungen an tierexperimentellen Modellen nachgewiesen werden. Die in dem nachstehend aufgeführten Modell untersuchten erfindungsgemäßen Verbindungen sind mit Nummern versehen worden, die den Nummern dieser Verbindungen in den Beispielen entsprechen.

### Prüfung der sekretionshemmenden Wirkung am perfundierten Rattenmagen

In der folgenden Tabelle A ist der Einfluß der erfindungsgemäßen Verbindungen nach intravenöser Gabe auf die durch Pentagastrin stimulierte Säuresekretion des perfundierten Rattenmagens in vivo dargestellt.

**Tabelle A**

| **Nr.** | **Dosis (µmol/kg) i.v.** | **Hemmung der Säureausscheidung (%)** |
|---|---|---|
| 9 | 3 | 100 |
| 10 | 3 | 100 |
| 11 | 3 | 100 |

### Methodik

Narkotisierten Ratten (CD-Ratte, weiblich, 200-250 g; 1,5 g/kg i.m. Urethan) wurde nach Tracheotomie das Abdomen durch einen medianen Oberbauchschnitt eröffnet und ein PVC-Katheter transoral im Osophagus sowie ein weiterer via Pylorus derart fixiert, daß die Schlauchenden eben noch in das Magenlumen hineinragten. Der aus dem Pylorus führende Katheter führte über eine seitliche Öffnung in der rechten Bauchwand nach außen.

Nach gründlicher Spülung (ca. 50-100 ml) wurde der Magen mit 37°C warmer physiologischer NaCl-Lösung kontinuierlich durchströmt (0,5 ml/min, pH 6,8-6,9; Braun-Unita I). In dem jeweils im 15 Minutenabstand aufgefangenen Effluat wurde der pH-Wert (pH-Meter 632, Glaselektrode EA 147; φ = 5 mm, Metrohm) sowie durch Titration mit einer frisch zubereiteten 0,01 N NaOH bis pH 7 (Dosimat 665 Metrohm) die sezernierte HCl bestimmt. Die Stimulation der Magensekretion erfolgte durch Dauerinfusion von 1 µg/kg (= 1,65 ml/h) i.v. Pentagastrin (V. fern. sin.) ca. 30 min nach Operationsende (d.h. nach Bestimmung von 2 Vorfraktionen). Die zu prüfenden Substanzen wurden intravenös in 1 ml/kg Flüssigkeitsvolumen 60 min nach Beginn der Pentagastrin-Dauerinfusion verabreicht.

Die Körpertemperatur der Tiere wurde durch Infrarot-Bestrahlung und Heizkissen (automatische, stufenlose Regelung über rektalen Temperaturfühler) auf konstant 37,8-38°C gehalten.

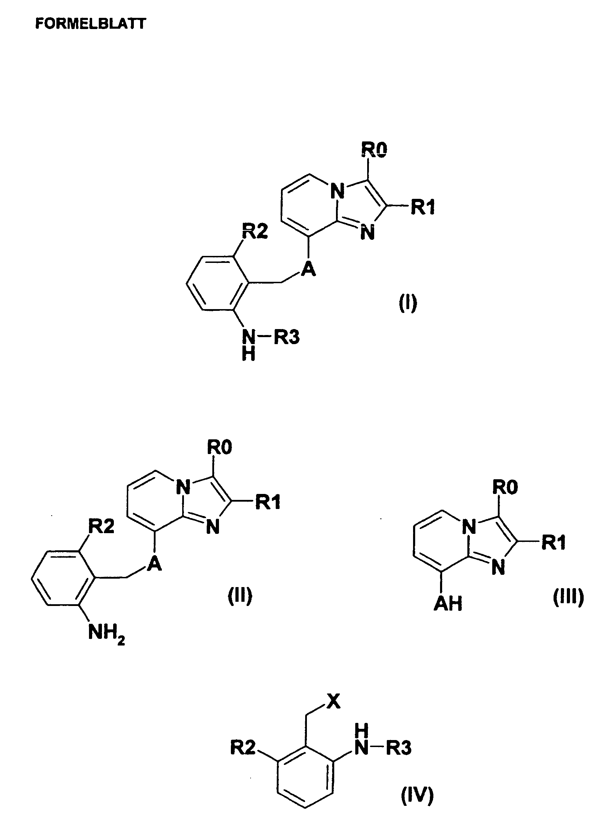

## Patentansprüche

1. Verbindungen der Formel I, 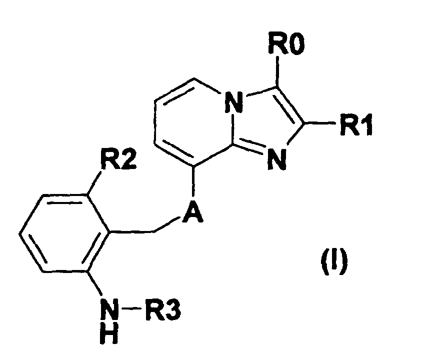 worin
R0 1-4C-Alkyl bedeutet,
R1 1-4C-Alkyl bedeutet,
R2 1-4C-Alkyl bedeutet,
R3 SO₂-R6,CO-R7 oder COO-R8 bedeutet,
A NH bedeutet,
R6 1-4C-Alkyl bedeutet,
R7 durch R16 substituiertes Phenyl bedeutet,
R8 durch R11 substituiertes 2-4C-Alkyl, durch R12 substituiertes 1-4C-Alkyl, -N=C(R13)R14 oder durch R15 substituiertes 2-4C-Alkyl bedeutet,
R11 1-4C-Alkoxy-1-4C-alkoxy bedeutet.
R12 1-4C-Alkoxycarbonyl, Carboxy, Thienyl, Pyridyl oder durch R16 substituiertes Phenyl bedeutet,
R13 1-4C-Alkyl und
R14 1-4C-Alkyl bedeuten,
R15 Phthalimidyl oder -S(O)ₙ-R19 bedeutet, wobei
R16 Hydroxy oder Benzoyl bedeutet,
R19 1-4C-Alkyl bedeutet und
n die Zahlen 0, 1 oder 2 bedeutet,
und ihre Salze.

2. Verbindungen der Formel I nach Anspruch 1, worin
R0 1-4C-Alkyl bedeutet,
R1 1-4C-Alkyl bedeutet,
R2 1-4C-Alkyl bedeutet,
R3 COO-R8 bedeutet,
A NH bedeutet,
R8 durch R11 substituiertes 2-4C-Alkyl, durch R12 substituiertes 1-4C-Alkyl, -N=C(R13)R14 oder durch R15 substituiertes 2-4C-Alkyl bedeutet,
R11 1-4C-Alkoxy-1-4C-alkoxy bedeutet,
R12 1-4C-Alkoxycarbonyl, Carboxy, Thienyl, Pyridyl oder durch R16 substituiertes Phenyl bedeutet,
R13 1-4C-Alkyl und
R14 1-4C-Alkyl bedeuten,
R15 -S(O)ₙ-R19 bedeutet, wobei
R16 Hydroxy bedeutet,
R19 1-4C-Alkyl bedeutet,
n die Zahlen 0,1 oder 2 bedeutet,
und ihre Salze.

3. Arzneimittel enthaltend eine Verbindung nach Anspruch 1 und/oder ein pharmakologisch verträgliches Salz davon zusammen mit üblichen pharmazeutischen Hilfs- und/oder Trägerstoffen.

4. Verbindungen nach Anspruch 1 und ihre pharmakologisch verträglichen Salze zur Anwendung bei der Verhütung und Behandlung gastrointestinaler Krankheiten.

5. Verwendung von Verbindungen nach Anspruch 1 und ihren pharmakologisch verträglichen Salzen zur Herstellung von Arzneimitteln für die Verhütung und Behandlung gastrointestinaler Krankheiten.

## Claims

1. Compounds of the formula I 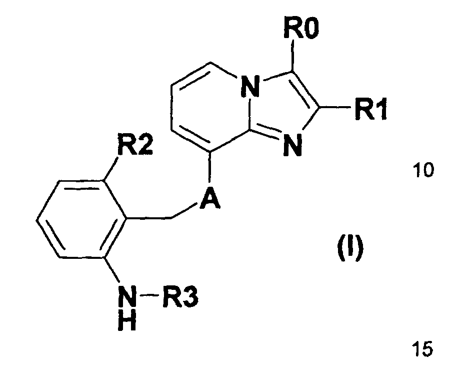 in which
R0 is 1-4C-alkyl,
R1 is 1-4C-alkyl,
R2 is 1-4C-alkyl,
R3 is SO₂-R6, CO-R7 or COO-R8,
A is NH,
R6 is 1-4C-alkyl,
R7 is phenyl substituted by R16,
R8 is 2-4C-alkyl substituted by R11, 1-4C-alkyl substituted by R12, -N=C(R13)R14 or 2-4C-alkyl substituted by R15,
R11 is 1-4C-alkoxy-1-4C-alkoxy,
R12 is 1-4C-alkoxycarbonyl, carboxyl, thienyl, pyridyl or phenyl substituted by R16,
R13 is 1-4C-alkyl and
R14 is 1-4C-alkyl,
R15 is phthalimidyl or -S(O)ₙ-R19, where
R16 is hydroxyl or benzoyl,
R19 is 1-4C-alkyl,
n is the numbers 0, 1 or 2,
and their salts.

2. Compounds of the formula 1 according to claim 1, in which
R0 is 1-4C-alkyl,
R1 is 1-4C-alkyl,
R2 is 1-4C-alkyl,
R3 is COO-R8,
A is NH,
R8 is 2-4C-alkyl substituted by R11, 1-4C-alkyl substituted by R12, -N=C(R13)R14 or 2-4C-alkyl substituted by R15,
R11 is 1-4C-alkoxy-1-4C-alkoxy,
R12 is 1-4C-alkoxycarbonyl, carboxyl, thienyl, pyridyl or phenyl substituted by R16,
R13 is 1-4C-alkyl and
R14 is 1-4C-alkyl,
R15 is -S(O)ₙ-R19, where
R16 is hydroxyl,
R19 is 1-4C-alkyl,
n is the numbers 0, 1 or 2.
and their salts.

3. Medicaments comprising a compound according to claim 1 and/or a pharmacologically tolerable salt thereof together with customary pharmaceutical auxiliaries and/or excipients.

4. Compounds according to claim 1 and their pharmacologically tolerable salts for use in the prevention and treatment of gastrointestinal diseases.

5. Use of compounds according to claim 1 and their pharmacologically tolerable salts for the production of medicaments for the prevention and treatment of gastrointestinal diseases.

## Revendications

1. Composés de formule I, 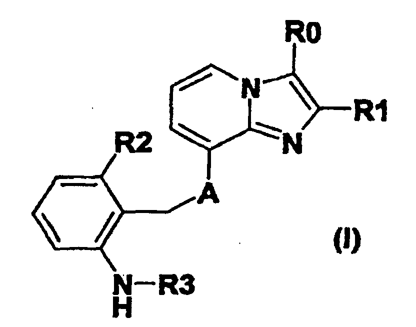 dans laquelle
R0 représente un alkyle de 1-4C,
R1 représente un alkyle de 1-4C,
R2 représente un alkyle de 1-4C,
R3 représente SO₂-R6, CO-R7 ou COO-R8,
A représente NH,
R6 représente un alkyle de 1-4C,
R7 représente un phényle substitué par R16,
R8 représente un alkyle de 2-4C substitué par R11, un alkyle de 1-4C substitué par R12, -N=C(R13)R14 ou un alkyle de 2-4C substitué par R15,
R11 représente un (alcoxy de 1-4C)-(alcoxy de 1-4C),
R12 représente un (alcoxy de 1-4C)carbonyle, carboxy, thiényle, pyridyle ou phényle substitué par R16,
R13 représente un alkyle de 1-4C et
R14 représente un alkyle de 1-4C,
R15 représente un phtalimidyle ou -S(O)ₙ-R19, dans lesquelles
R16 représente un hydroxy ou benzoyle,
R19 représente un alkyle de 1-4C et
n représente les nombres 0, 1 ou 2,
et leurs sels.

2. Composés de formule I selon la revendication 1, dans lesquels
R0 représente un alkyle de 1-4C,
R1 représente un alkyle de 1-4C,
R2 représente un alkyle de 1-4C,
R3 représente COO-R8,
A représente NH,
R8 représente un alkyle de 2-4C substitué par R11, un alkyle de 1-4C substitué par R12, -N=C(R13)R14 ou un alkyle à 2-4C substitué par R15,
R11 représente un (alcoxy de 1-4C)-(alcoxy de 1-4C),
R12 représente un (alcoxy de 1-4C)carbonyle, carboxy, thiényle, pyridyle ou phényle substitué par R16,
R13 représente un alkyle de 1-4C et
R14 représente un alkyle de 1-4C,
R15 représente-S(O)ₙ-R19, dans lesquelles
R16 représente un hydroxy,
R19 représente un alkyle de 1-4C et
n représente les nombres 0, 1 ou 2,
et leurs sels.

3. Médicament contenant un composé selon la revendication 1 et/ou un sel pharmaceutiquement acceptable de celui-ci en même temps que des adjuvants et/ou additifs pharmaceutiques usuels.

4. Composés selon la revendication 1 et leurs sels pharmaceutiquement acceptables destinés à une utilisation pour la prévention et le traitement de maladies gastro-intestinales.

5. Utilisation de composés selon la revendication 1 et de leurs sels pharmaceutiquement acceptables pour la préparation de médicaments pour la prévention et le traitement de maladies gastro-intestinales.
